(19) Europäisches Patentamt
European Patent Office
Office européen des brevets

(11) **EP 4 666 956 A1**

(12) **EUROPEAN PATENT APPLICATION**

(43) Date of publication:
**24.12.2025 Bulletin 2025/52**

(21) Application number: 24198482.2

(22) Date of filing: **04.09.2024**

(51) International Patent Classification (IPC):
**A61B 8/00** *(2006.01)*    **G01S 7/52** *(2006.01)*

(52) Cooperative Patent Classification (CPC):
**A61B 8/488; A61B 8/06; A61B 8/0891;
A61B 8/465; A61B 8/469; G01S 7/52073**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.06.2024 US 202463662472 P**

(71) Applicant: **Koninklijke Philips N.V.
5656 AG Eindhoven (NL)**

(72) Inventors:
• **YU, Jason Richard
Eindhoven (NL)**

• **BARNHART, Krislynn
Eindhoven (NL)**
• **LOUPAS, Thanasis
5656 AG Eindhoven (NL)**
• **PITTS, Conner David
Eindhoven (NL)**
• **WIGEN, Jason
Eindhoven (NL)**

(74) Representative: **Philips Intellectual Property &
Standards
High Tech Campus 52
5656 AG Eindhoven (NL)**

(54) **ULTRASOUND IMAGING SYSTEM USER INTERFACE FOR SELECTING A VESSEL OF INTEREST**

(57)    An ultrasound system operable to perform a color Doppler exam includes a graphic watermark (64) linked to move in synchronism with a color box (62) over a color Doppler image. The user can reposition the color box and watermark over the image so that the watermark designates which blood vessel of a plurality of blood vessels in the image is the vessel of interest (1) for the exam. Preferably the color box is repositioned with an image control that is frequently used during the workflow of a color Doppler exam, such as a trackball. In response to the designation of a blood vessel of interest for diagnosis, the ultrasound system automatically configures color Doppler imaging parameters for the blood vessel of interest.

FIG. 4

**EP 4 666 956 A1**

**Description**

FIELD OF THE INVENTION

This invention relates to medical diagnostic ultrasound systems and, in particular, to ultrasound systems with a user interface which enables selection of a vessel of interest without interrupting standard diagnostic workflow.

BACKGROUND OF THE INVENTION

Performing an ultrasonic diagnostic imaging exam with an ultrasound system requires not only extensive medical expertise. It also requires the sonographer to perform several actions simultaneously. In the typical diagnostic exam, the sonographer is pressing an ultrasound probe against the body of the patient with one hand and manipulating controls on the ultrasound system with the other hand while keeping her eyes glued to the display screen so as to detect the anatomy of interest in the ultrasound image. The ultrasound system does not image optically, of course. Its function is to transmit ultrasound waves into the body, receive acoustic echoes from those transmissions, and process the acoustic echoes to produce images of the structure and functioning of internal anatomy. The process of producing the required ultrasound waves and converting the resulting echoes into the desired image information is complex and subject to the influence of many variables. Among these are the intensity, frequency and steering of the transmitted waves, the reception and sampling of the received echo information, the beamformation, filtering and demodulation of the received echo signals, and the extensive signal processing techniques used to convert the echo signals into images of tissue, blood flow, or parameters of the condition of tissue and/or blood flow. The upshot of all of this complexity is that an ultrasound system has a large number of controls and control parameters which must be set and adjusted by the sonographer in order to acquire the desired diagnostic images. And it must be kept in mind that the sonographer is operating the ultrasound system with only one hand while focusing her attention on the appearance of the internal anatomy on the display screen. In sum, she is operating the ultrasound system with one hand, blindly.

In order to ease the inherent difficulty of performing this feat, modern ultrasound systems are programmed with numerous algorithms that are directed to controlling the operation of the ultrasound system automatically. Many of these algorithms are adaptive in nature, automatically responding to the conduct of the exam and its received images. For instance, if the sonographer begins imaging at a deeper depth in the body, the system may respond with increased acoustic transmission and the processing of signals at lower frequencies for better image penetration at greater depths of imaging, such as occurs during a deep abdominal exam. In a vascular exam, the appearance of clutter in the image may cause a change in the color wall filter cutoff frequency to reduce artifacts from blood vessel walls, or a change to the tissue harmonic imaging mode. A vessel close to the ultrasound transducer may exhibit excessive color bleeding in the color Doppler mode, the response to which may be a reduction of the color gain and/or an increase in the grayscale priority (color write priority) setting. Correspondingly, if the sonographer moves to image a deeper vessel which exhibits poor color filling, the response may be to increase the color gain or decrease the color scale and/or color transmit frequency. Thus, an ultrasound system is constantly trying to adapt its performance to the dynamics of the ultrasound exam.

One situation which poses a dilemma for such adaptation is a vascular exam in which numerous blood vessels, including a vessel of interest, are present in the image. A typical ultrasound system will operate in a Doppler mode, such as color Doppler, pulse-wave Doppler, continuous wave Doppler or power Doppler, to assess the viability of blood flow in blood vessels. Since Doppler flow measurement requires repeated or continuous interrogation of the flow, which requires a relatively substantial number of transmissions and receptions for 2D or 3D Doppler imaging, the typical ultrasound system will enable the sonographer to position a graphic "color box" over the ultrasound image. Doppler interrogation will then be performed on the anatomy inside the color box in the image and not on either side of the box, thereby reducing the number of transmission-reception cycles needed to form an image and thus improving the frame rate of display. But when many vessels in the image are positioned in close proximity to each other and are located in the color box, the automation algorithms face a dilemma: which vessel is the vessel of interest, for which the performance of the ultrasound system should be optimized? The sonographer could end this dilemma by designating the vessel of interest by clicking on it with a system pointing device or using a finger to touch the vessel of interest on a touchscreen display. The problems with these approaches are that they require additional hand movements by the sonographer and disrupt the regular scanning workflow. Accordingly, a solution is needed which solves the dilemma of identifying the vessel of interest without disrupting the sonographer's regular scanning workflow.

SUMMARY OF THE INVENTION

The invention is defined by the independent claims. The dependent claims define advantageous embodiments.
In accordance with embodiments of the present invention, an ultrasound system is provided with the capability of performing a vascular exam. The ultrasound system display includes a user-positionable graphic color box which may be positioned over an ultrasound image to designate a region in which Doppler ultrasound imaging is performed. The color box includes a graphic icon, preferably at the center of the box, which moves with the box as the position of the color box is adjusted by a user. When multiple vessels are located in the color box, the user positions the color box so that the graphic icon is positioned over a vessel of interest or in close proximity to the vessel of interest. This may be done simply with the trackball of the typical ultrasound system, over which the hand of the sonographer is generally positioned during an exam. The sonographer rolls the trackball with her fingertips until the color box is positioned with its graphic icon over or closely adjacent to the vessel of interest. The ultrasound system responds to this positioning by controlling one or more system operating parameters to be optimal for diagnosis of the indicated vessel of interest.

According to an aspect there is provided a computer-implemented method of processing an ultrasound image, the method comprising:

- receiving an ultrasound image comprising at least one blood vessel;
- generating a user-movable color box in the ultrasound image;
- generating a user-movable watermark within the user-movable color box in the ultrasound image; and
- designating a blood vessel of interest from the at least one blood vessel based on a proximity of the watermark to one of the blood vessels.

In this manner, a user of an ultrasound system may easily select a singular blood vessel of interest, even when multiple blood vessels are present within a user-movable color box.

In an example, the method further comprises:

- adjusting an adaptive imaging system parameter based on the designated blood vessel of interest. The adaptive imaging system parameter may be adjusted to optimize a display of the designated blood vessel of interest in an ultrasound image.

In an example, the user-movable color box and the user-movable watermark are user-movable in synchronism.

In an example, the adaptive imaging system parameter comprise color Doppler imaging parameters, wherein the color Doppler imaging parameters are preferably any one or a combination of: transmit Frequency, Color Box Steering, Color Scale, Gain, Frequency, Wall Filter, Persistence, Smoothing, Dynamic Range and Grayscale Priority.

In an example, the method further comprises:

- receiving a user input to move the user-movable color box and/or the user-movable watermark on the ultrasound image.

According to an aspect, there is provided a computer program product comprising instructions, which when carried out by a processor, cause the processor to carry out any herein described method.

According to an aspect there is provided an ultrasonic diagnostic imaging system comprising a processor configured to carry out any herein described method.

According to an aspect there is provided an ultrasonic diagnostic imaging system operable to perform a Doppler ultrasound exam, the system comprising:

    an ultrasound probe adapted to acquire Doppler ultrasound image signals;
    a Doppler processor (34) coupled to the ultrasound probe, and adapted to generate a color Doppler image of two or more blood vessels;
    a graphics processor (46) adapted to generate a watermark movably linked to a color box for an image display;
    an image display (40) coupled to the Doppler processor and the graphics processor, and adapted to display the color Doppler image and the watermark movably linked to the color box; and
    a user interface (38) responsive to a user input and adapted to reposition the watermark in relation to the blood vessels.

According to an aspect there is provided an ultrasonic diagnostic imaging system operable to perform a Doppler ultrasound exam, the system comprising:

    an ultrasound probe adapted to acquire Doppler ultrasound image signals;
    a Doppler processor coupled to the ultrasound probe, and adapted to produce signals for a Doppler image;
    an image processor coupled to the Doppler processor, and adapted to produce Doppler image signals of a plurality of blood vessels;
    a graphics processor adapted to produce a color box with a watermark for an image display;
    an image display coupled to the image processor and the graphics processor, and adapted to display a Doppler image with a color box having a watermark, the color box encompassing Doppler images of the plurality of blood vessels; and
    a user interface that, in response to user operation, is adapted to control the position of the color box and the watermark on the display so that the position of the watermark designates a blood vessel of interest for diagnosis.

In an example, the system further comprises one or more adaptive imaging system parameters that are optimized for the designated blood vessel of interest. The adaptive imaging system parameter may be adjusted to optimize the display of the designated blood vessel of interest in a Color Doppler image.

In an example, the one or more adaptive imaging system parameters comprise color Doppler imaging parameters.

In an example, an adaptive imaging system parameter which is optimized in response to designation of a blood vessel of interest comprises transmit Frequency.

In an example, an adaptive imaging system parameter which is optimized in response to designation of a blood vessel of interest comprises Color Box Steering.

In an example, the one or more adaptive imaging system parameters further comprise one or more of the parameters Color Scale, Gain, Frequency, Wall Filter, Persistence, Smoothing, Dynamic Range, Grayscale Priority, Color Box Position, and Color Box Steering.

In an example, the user interface further comprises a user control adapted to position the color box and watermark and that is commonly used during the workflow of a Doppler ultrasound exam.

In an example, the user control further comprises a trackball located on an ultrasound system control panel.

In an example, the watermark further comprises a graphic symbol such as an "O", an "X" or a "+" sign.

In an example, the color box and the watermark move in synchronism in response to user operation of a user control of the user interface.

According to an aspect of the invention there is provided an ultrasonic diagnostic imaging system operable to perform a Doppler ultrasound exam comprising:

> an ultrasound probe adapted to acquire Doppler ultrasound image signals;
> a Doppler processor coupled to the ultrasound probe, and adapted to generate a color Doppler image of two or more blood vessels;
> a graphics processor adapted to generate a watermark movably linked to a color box for an image display;
> an image display coupled to the Doppler processor and the graphics processor, and adapted to display the color Doppler image and the watermark movably linked to the color box; and
> a user interface responsive to a user input and adapted to reposition the watermark in relation to the blood vessels.

In an example, the system further comprises a processor adapted to automatically configure color imaging parameters based on a proximity of the watermark to one of the blood vessels.

In an example the processor is adapted to automatically configure color imaging parameters further comprises one or more of a beamformer controller, a signal processor, a Doppler processor, a graphics processor and an image processor.

In an example, the graphics processor is further adapted to generate a secondary watermark visually indicating a blood vessel of interest for a Doppler exam.

In an example, the secondary watermark further comprises a tracing of a blood vessel of interest or a numbering of a blood vessel of interest.

These and other aspects of the invention will be apparent from and elucidated with reference to the embodiments described herein.

BRIEF DESCRIPTION OF THE DRAWINGS

FIGS 1 and 2 illustrate ultrasound images with multiple blood vessels with different flow conditions inside the color boxes in the images.

FIG. 3 illustrates an ultrasound image with a graphic color box having a graphic icon in the center in accordance with the principles of the present invention.

FIG. 4 illustrates the ultrasound image of FIGURE 3 in which the color box has been repositioned by a user so that the graphic icon is closest to the vessel of interest in the image.

FIG. 5 illustrates the ultrasound image of FIGURE 4 in which the vessels shown in the image have additionally been numerically designated.

FIG. 6a illustrates the identified vessel of interest of FIGURE 5 in enlarged detail.

FIG. 6b illustrates the vessel of interest being identified to the user by a border tracing.

FIG. 7 illustrates in block diagram form an ultrasound system constructed in accordance with the principles of the present invention.

FIG. 8 illustrates in block diagram form the color Doppler processor of FIGURE 7 in greater detail.

DESCRIPTION OF THE EMBODIMENTS

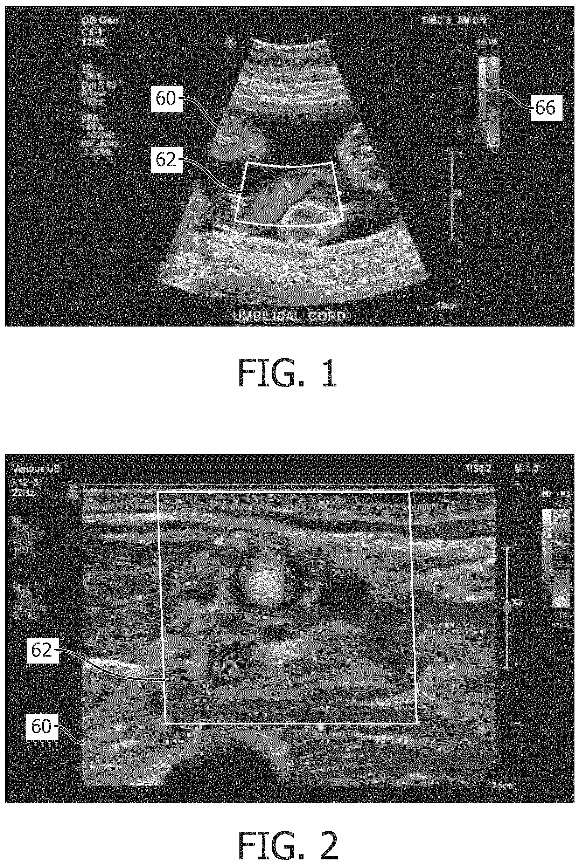

FIG. 1 illustrates a color Doppler ultrasound image 60 of a developing fetus in the uterus of the mother which is produced using a curved linear array transducer probe. A color box 62 is located over the umbilical cord in the image. Doppler interrogation and processing are performed at locations of the anatomy within the color box, and the movement of fluids within the color box will result in Doppler signal returns that are depicted in color. The range of colors and their corresponding velocity and direction representations are shown in the color bar 66 to the right of the image. One range of colors (e.g., blues) represent flow in one direction with respect to the transducer probe (e.g., toward the transducer). Another range of colors (e.g., reds) represent flow in the other direction (e.g., away from the transducer). The alternating shades of color of the umbilical cord in the color box 62 are produced from the twisted blood vessels of arterial and venous flow to and from the fetus. Which is arterial and which is venous flow may be determined by the velocity and timing of flow in relation to the mother's ECG signal. In a typical obstetrical exam, the sonographer may be measuring the velocity or volume of blood flow in a selected one of the vessels, for instance. The flow of one of the vessels displayed in color box 62 may be selected for diagnosis in accordance with the present invention.

FIG. 2 illustrates a lower extremities ultrasound image 60 produced by a linear array transducer probe. The image plane of the probe is seen to intersect a number of blood vessels which are shown in the color box 62 as generally circular regions of differing shades of reds and blues. Any one of these vessels may be selected as the vessel of interest for flow analysis in accordance with the present invention.

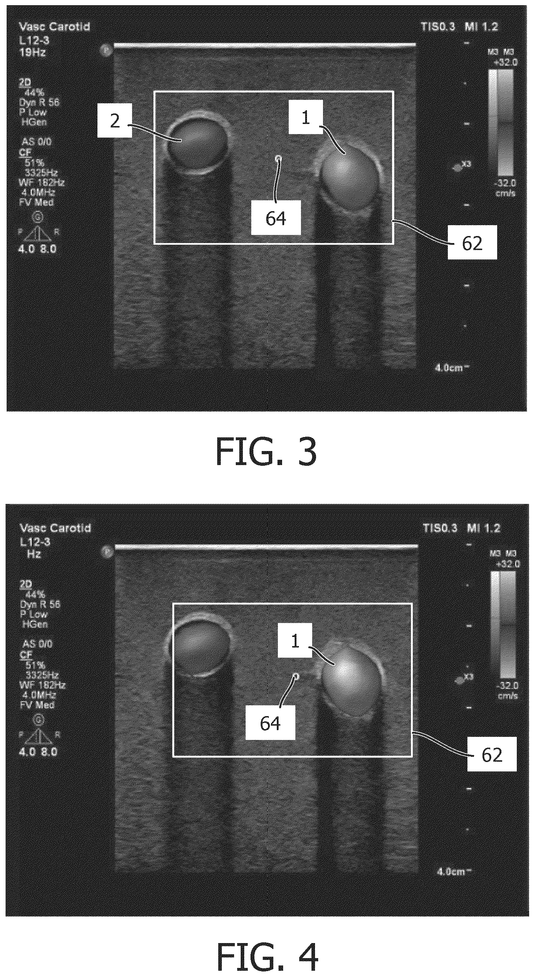

FIG. 3 illustrates an image plane acquired by a linear array probe which intersects a simulated carotid artery and jugular vein in an ultrasound phantom. The large color areas in the image illustrate the arterial flow 1 and the venous flow 2 of the vessels. A color box 62 is located around the flow regions so that the flow in the vessels will be Doppler-processed and displayed. In accordance with the principles of the present invention, the graphic color box 62 also includes a graphic icon 64 which moves in synchronism with movement of the color box by the sonographer. In this example the graphic icon 64, referred to herein as a "watermark," is depicted as a circle, although it can be any other shape or symbol such as an "X" or a "+". Other possible watermarks include text labels and computer graphic effects such as transparency, scintillation, and hue changes. The watermark 64 is preferably located at the center of the color box 62. In this starting position for the color box in FIGURE 3, the watermark 64 is roughly located intermediate the arterial flow 1 and the vascular flow 2.

In accordance with the principles of the present invention, the sonographer moves the color box 62 with its watermark 64 so that the watermark is positioned over or close to a vessel of interest which is to be diagnosed. The sonographer does this by manipulating a control on the user control panel that is in active use during the current workflow of the exam. An example of such a control is the trackball on the control panel, over which a hand of the sonographer generally hovers during significant periods of an exam. A trackball user control is commonly used during the workflow of a Doppler ultrasound exam. The sonographer can quickly find and manipulate the trackball with one hand, without taking her eyes off of the ultrasound image illustrated in FIGURE 4, and can easily reposition the color box and its synchronized watermark so that the watermark 64 is on or adjacent to the intended vessel of interest. As FIGURE 4 illustrates, in this example the sonographer has repositioned the watermark 64 from its initial intermediate position shown in FIGURE 3 to a position where it is closest to the flow of arterial vessel 1 as shown in FIGURE 4. The close proximity of the watermark 64 to vessel 1 informs the ultrasound system that vessel 1 is the vessel of interest, and the automated Doppler optimization controls proceed to optimize the imaging of vessel 1 for a precise diagnosis. In other words, the system will adjust adaptive imaging system parameters to optimize imaging for the blood vessel of interest. Positioning the watermark near the vessel of interest also locates the vessel centrally in the box, which is preferable for reducing boundary effects.

A typical ultrasound system generally has numerous controls (i.e., adaptive imaging system parameters) which can be automatically and adaptively set for a Doppler flow diagnostic procedure. Among these are Color Scale, Gain, Frequency, Wall Filter, Persistence, Smoothing, Dynamic Range, Grayscale Priority, Color Box Position, Color Box Steering, PW Doppler Sample Volume Position, PW Doppler Steering, PW Doppler Angle Correction, PW Doppler Gain, PW Doppler Scale, and PW Doppler Baseline Invert. In an implementation of the present invention, one or more settings such as these are adjusted to best optimize a Doppler image for the vessel indicated by the location of the color box watermark. For instance, if the selected vessel of interest is more toward the top of the image, the system may respond by increasing the transmit Frequency of the Doppler beam for better resolution of a shallow vessel. If the selected vessel is more toward a side of the image, the system may respond by adjusting the Color Box Steering (beam angle) or the Doppler Angle Correction for more efficient angle correction. If the selected vessel exhibits quick, momentary peak flow velocities, greater Persistence may be employed. Other useful responses to the characteristics of a selected vessel and its flow will be readily apparent to those skilled in the art.

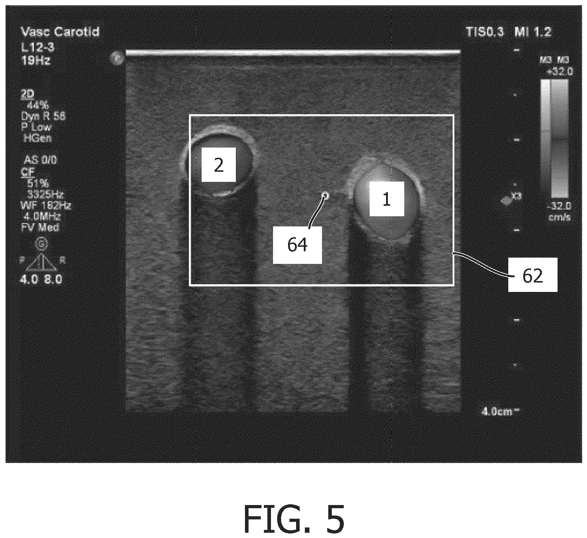

Alternatively or in addition to the optimization of Doppler settings for a selected vessel, an ultrasound system of the present invention may display a secondary watermark, in addition to the primary watermark, to graphically identify vessels detected in an image. This is illustrated in FIGURE 5, where it is seen that the vessel on the right, the one closest to the primary vessel selection watermark 64, has been identified as vessel #1, and the vessel to the left in the image has been identified as vessel #2. The graphic characters 1 and 2 comprise secondary watermarks in this example. Corresponding lines of text on the display may further state characteristics of vessels #1 and #2, such as:

    Carotid artery
    Jugular vein

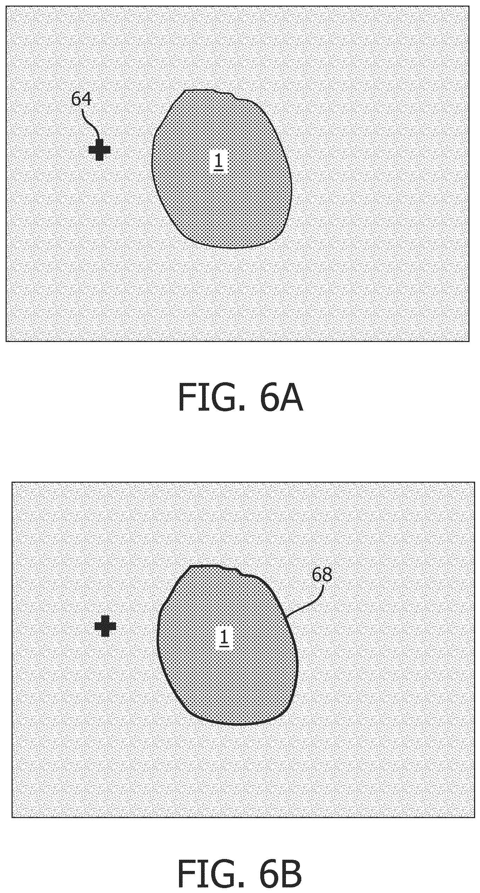

Another way for the ultrasound system to indicate to the sonographer with secondary watermarks that a vessel has been identified as a vessel of interest is illustrated in FIGURES 6a and 6b. FIGURE 6a illustrates the identified vessel of interest of FIGURE 5 in an enlarged view with the vessel identified by the numeral 1. The primary watermark 64, here illustrated as a "+" sign, is seen positioned just to the left of the identified vessel 1. In FIGURE 6b, the border of vessel 1 has been traced with a heavy border tracing 68, visually indicating to the sonographer that vessel # 1 is the identified vessel of interest for the ultrasound exam. The border tracing 68 is the secondary watermark in this example.

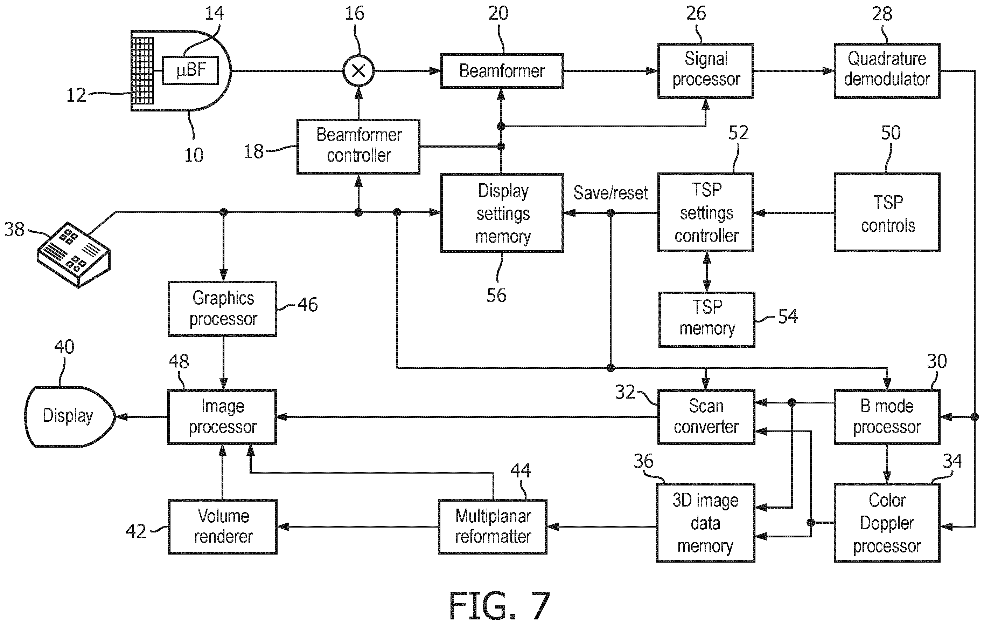

Referring now to FIGURE 7, an ultrasonic diagnostic imaging system constructed in accordance with the principles of the present invention is shown in block diagram form. A transducer array 12 is provided in an ultrasound probe 10 for transmitting ultrasonic waves and receiving echo information. The transducer array 12 may be a one- or two-dimensional array of transducer elements capable of scanning in two or three dimensions, for instance, in both elevation (in 3D) and azimuth. In this example of a 3D imaging probe, the transducer array 12 is a two-dimensional array coupled to a microbeamformer 14 in the probe which controls transmission and reception of signals by the array elements. A microbeamformer is generally not used when the probe has a one-dimensional array for 2D imaging. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main beamformer 20 from high energy transmit signals. The transmission of ultrasonic beams from the transducer array 12 under control of the microbeamformer 14 is directed by a beamformer controller 18 coupled to the T/R switch and the beamformer 20, which receives input from the user's operation of the user interface or control panel 38. Among the transmit characteristics controlled by the beamformer controller are the frequency, steering, number, spacing, amplitude, phase, and polarity of transmit and receive beams. Beams formed in the direction of pulse transmission may be steered straight ahead from the transducer array, or at different angles for a wider sector field of view or better Doppler reception.

The echoes received by a contiguous group of transducer elements are beamformed by appropriately delaying them and then combining them. The partially beamformed signals produced by the microbeamformer 14 from each patch of elements are coupled to a main beamformer 20 where partially beamformed signals from individual patches of transducer elements are combined into a fully beamformed coherent echo signal. For example, the main beamformer 20 may have 128 channels, each of which receives a partially beamformed signal from a patch of 12 transducer elements. In this way the signals received by over 1500 transducer elements of a two-dimensional array transducer can contribute efficiently to a single beamformed signal.

The coherent echo signals undergo signal processing by a signal processor 26, which includes filtering by a digital filter and noise reduction as by spatial or frequency compounding. The signal processor can also shift the frequency band to a lower or baseband frequency range. The digital filter of the signal processor 26 can be a filter of the type disclosed in U.S. Patent No. 5,833,613 (Averkiou et al.), for example. The processed echo signals then are demodulated by a quadrature demodulator 28 into quadrature (I and Q) components, which provide signal phase information for Doppler processing.

The beamformed and processed coherent echo signals are coupled to a B-mode processor 30 which produces a B-mode image of structure in the body such as tissue. The B-mode processor performs amplitude (envelope) detection of quadrature demodulated I and Q signal components by calculating the echo signal amplitude in the form of $(I^2+Q^2)^{1/2}$. The quadrature echo signal components are also coupled to a Doppler processor 34. The Doppler processor 34 stores ensembles of echo signals from discrete points in an image field which are then used to estimate the Doppler shift at points in the image, typically by means of a fast Fourier transform (FFT) processor or a lag-one autocorrelation technique. The rate at which the ensembles are acquired determines the velocity range of motion that the system can accurately measure and depict in an image. The Doppler shift is proportional to motion at points in the image field, *e.g.,* blood flow and tissue motion. For a color Doppler image, the I and Q values of an ensemble are wall filtered prior to the estimation of the mean Doppler frequency or power. The wall filter has an adjustable cutoff frequency above or below which motion will be rejected such as the low frequency motion of the wall of a blood vessel when imaging flowing blood. The B-mode image signals and the Doppler flow values are coupled to a scan converter 32 which converts the B-mode and Doppler samples from their acquired R-$\theta$ coordinates to Cartesian (x,y) coordinates for display in a desired display format, e.g., a rectilinear display format or a sector display format. Either the B-mode image or the Doppler image may be displayed alone, or the two shown together in anatomical registration in which the color Doppler overlay shows the blood flow in tissue and vessels in the B-mode image as shown in FIGURES 1-5. Another display possibility is to display side-by-side images of the same anatomy which have been processed differently. This display format is useful when comparing images.

The image data produced by the B-mode processor 30 and the Doppler processor 34 are coupled to a 3D image data memory 36, where it is stored in memory locations addressable in accordance with the spatial locations from which the image values were acquired. A multiplanar reformatter 44 converts echoes which are received from points in a common plane in a volumetric region of the body into an ultrasonic image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.) The 3D images and 2D images produced by the scan converter 32, the multiplanar reformatter 44, and the volume renderer 42 are coupled to an image processor 48 for further enhancement, buffering and temporary storage for display on an image display 40. A graphic display overlay containing textual, parametric, and other graphic information such as patient ID and color box with a watermark is produced by a graphics processor 46 and coupled to the image processor for display with the ultrasound images.

The implementation of FIG. 7 also includes tissue specific preset (TSP) controls 50 on the user interface 38 or a touchscreen control panel by which the sonographer may recall control parameter settings (e.g., adaptive imaging system parameters) from memory to initialize an exam. A typical ultrasound system is manufactured with factory installed presets for particular types of exams such as vascular, abdominal, or obstetrical exams. These presets may be recalled from memory by the sonographer to set up the ultrasound system for a particular exam type. Sonographers are also able to store settings from their previous exams and recall them for future exams. See US Pat. 5,315,999 (Kinicki et al.) and US Pat. 11,744,557 (Holmes et al.) for further details on exam presets and their use. Using the TSP controls 50, the sonographer can command a TSP settings controller 52 to recall a desired set of presets for an exam from a TSP memory 54. The TSP settings controller then applies the selected presets to processors and other devices of the ultrasound system, conditioning them for the desired exam. A number of the presets are saved in a display settings memory, and used to set up display parameters such as the depth of images, display format (e.g., sector or rectilinear), and focal depths. Color may be turned on or off. The settings in the display settings memory 56 are updated as the sonographer invokes changes during an exam, such as adjusting focal depths. The values in the display settings memory are re-initialized to default values when a TSP mode is changed.

In accordance with the principles of the present invention the user interface 38 includes a user control which is in frequent use during the workflow of a Doppler exam, such as the trackball shown in the lower right corner of the control panel 38. The trackball is generally in constant use during a color Doppler exam as the sonographer changes the color box position or size, positions the PW Doppler sample volume, selects different system option, and actuates graphic controls on the display screen. A hand of the sonographer will generally hover over the trackball as the sonographer uses the other hand to hold the transducer probe against the body of the patient while continually observing the anatomy and its orientation on the display screen. When a color box is called up for a Doppler exam, its position over the anatomical image can be adjusted by the trackball at the fingertips of the sonographer without diverting the sonographer's eyes from the image on the screen. A color box with a vessel-selecting watermark is thereby positioned so that the watermark is over or closest to a vessel of interest for diagnosis. The system responds by identifying the indicated vessel as by numbering it or outlining it as illustrated in FIGURES 6a and 6b. With the vessel of interest thereby recognized by the ultrasound system, the automated optimization algorithms of the system will then optimize the system, by adjusting adaptive imaging system parameters, for optimal imaging of the vessel of interest. The beamformer controller can automatically adjust characteristics such as transmit frequency or color pulse repetition frequency (PFR), which determines the color velocity scale. The Doppler beam angle and signal processing filter parameters can be optimized, the Doppler processor can adjust color gain and grayscale priority, the graphics processor can adjust the color box position or size, and the image processor can adjust color bar range and colors and display objects corresponding to other system adjustments. The sonographer can then proceed without interruption or delay to make an accurate assessment of the flow characteristics of the vessel of interest.

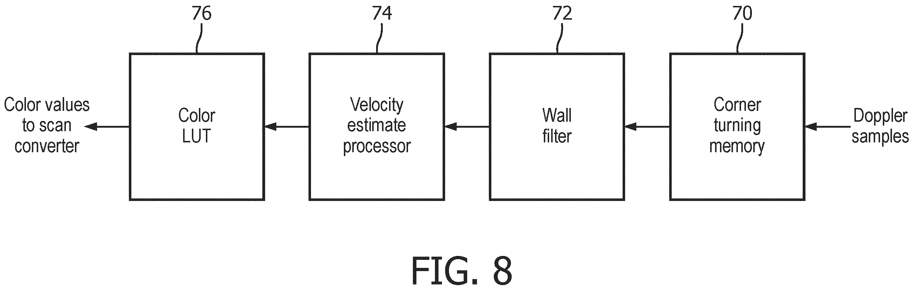

FIG. 8 is a more detailed illustration of a typical Doppler processor for an ultrasound system. The beamformer controller controls the sampling of points in an image field by repetitively acquiring echo signals from each image point in the anatomy in the color box at a predetermined frequency referred to as the pulse repetition frequency (PRF). The samples repetitively acquired from along a beam direction in the image field are stored in a corner turning memory 70. When one line of samples is acquired, the samples are stored in one direction (e.g., vertically) in the corner turning memory. When samples along the line are acquired by the next sampling pulse at the PRF rate, they are stored in the same direction next to the first line of samples. The corner turning memory is repetitively filled in this manner. Each row of samples in the orthogonal direction contains samples acquired at different times from the same point along the scan line. When Doppler shift values are to be calculated for the line, samples are read out in the orthogonal direction (e.g., horizontally) and processed by the velocity estimate processor 74, which uses a form of the Doppler equation to produce Doppler shift values at successively different depths along the line. When expressed in terms of frequency shift, the Doppler equation is:

$$\Delta f = (2f_0 V \, \mathrm{Cos}\theta)/c$$

where $\Delta f$ is the frequency shift, $f_0$ is the frequency of the transmitted wave, V is the velocity of the reflecting object (*e.g.*, a red blood cell), $\theta$ is the angle between the incident wave and the direction of the motion of the reflecting object (*i.e.*, the angle of incidence), and c is the velocity of sound in the medium. When processing ensemble samples for color Doppler display, a commercial ultrasound system will typically use a form of the Kasai one-lag autocorrelation algorithm.

Flow velocity estimates are produced in this manner for each location in the color box 62 where flow is occurring. For color Doppler displays such as those shown above, the ensemble values are wall filtered prior to Doppler processing by a wall filter 72, which eliminates artifacts from vessel wall motion when imaging blood flow, and artifacts from flow when imaging tissue motion. The Doppler flow estimates produced by the velocity estimate processor 74 are converted to a corresponding display color value by a color look-up table (LUT) 76, coupled to the scan converter or the 3D image data memory, and used for a pixel color on the image display. In the ultrasound images of FIGURES 3, 4, and 5, for example, it is seen that shades of red pixels fill vessel #1, indicating (according to the color bar) blood flow toward the transducer probe, and shades of blue pixels fill vessel #2, indicating flow away from the transducer.

It should be noted that an ultrasound system suitable for use in an implementation of the present invention, and in particular the component structure of the ultrasound system of FIG. 7, may be implemented in hardware, software, or a combination thereof. The various embodiments and/or components of an ultrasound system, for example, the beamformer controller, the graphics processor, and the Doppler processor or components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system or the data network for importing training images. The computer or processor may also include a memory. The memory devices such as the TSP memory 54, the corner turning memory 70, and the display settings memory 56 may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and display of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules such as a neural network model module, a program module within a larger program or a portion of a program module. The software also may include modular programming in the form of object-oriented programming. The processing of input data by the processing machine may be in response to operator commands, or in response to results of previous processing, or in response to a request made by another processing machine.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "coupled" does not necessarily mean directly coupled; so, if element A is coupled to element B, there may be intermediate elements between elements A and B. The word "a" or "an" preceding an element does not exclude the presence of a plurality of such elements. The invention may be implemented by means of hardware comprising several distinct elements, and/or by means of a suitably programmed processor. The computer program product may be software available for download from a server, e.g., via the internet. Alternatively, the computer program product may be a suitable (non-transitory) computer readable medium on which the instructions are stored, such as an optical storage medium or a solid-state medium, which may or may not be supplied together with or as part of other hardware. In the device claim enumerating several means, several of these means may be embodied by one and the same item of hardware. Measures recited in mutually different dependent claims may advantageously be used in combination.

## Claims

1. An ultrasonic diagnostic imaging system operable to perform a Doppler ultrasound exam, the system comprising:

an ultrasound probe (10) adapted to acquire Doppler ultrasound image signals;
a Doppler processor (34) coupled to the ultrasound probe, and adapted to generate a color Doppler image of two or more blood vessels;
a graphics processor (46) adapted to generate a watermark movably linked to a color box for an image display;
an image display (40) coupled to the Doppler processor and the graphics processor, and adapted to display the color Doppler image and the watermark movably linked to the color box; and
a user interface (38) responsive to a user input and adapted to reposition the watermark in relation to the blood vessels.

2. The ultrasonic diagnostic imaging system of Claim 1, further comprising adjusting an adaptive imaging system parameter based on the designated blood vessel of interest.

3. The ultrasonic diagnostic imaging system of Claim 1 or 2, wherein the adaptive imaging system parameters comprise color Doppler imaging parameters, wherein the color Doppler imaging parameters are preferably any one or a combination of: transmit Frequency, Color Box Steering, Color Scale, Gain, Frequency, Wall Filter, Persistence, Smoothing, Dynamic Range and Grayscale Priority.

4. The ultrasonic diagnostic imaging system of any one of Claims 1 to 3, wherein the user interface further comprises a user control adapted to position the color box and/or the watermark and that is commonly used during the workflow of a Doppler ultrasound exam.

5. The ultrasonic diagnostic imaging system of Claim 4, wherein the user control further comprises a trackball located on an ultrasound system control panel.

6. The ultrasonic diagnostic imaging system of any one of Claims 1 to 5, wherein the watermark further comprises a graphic symbol such as an "O", an "X" or a "+" sign.

7. The ultrasonic diagnostic imaging system of any one of Claims 1 to 6, wherein the color box and the watermark move in synchronism in response to user operation of a user control of the user interface.

8. The ultrasonic diagnostic imaging system of any one of Claims 1 to 7, wherein the blood vessel is designated based on a proximity of the watermark to one of the blood vessels.

9. The ultrasonic diagnostic imaging system of any one of Claims 1 to 8, wherein the graphics processor is further adapted to generate a secondary watermark visually indicating a blood vessel of interest for a Doppler exam, wherein optionally the secondary watermark further comprises a tracing of a blood vessel of interest or a numbering of a blood vessel of interest.

10. A computer-implemented method of processing an ultrasound image, the method comprising:

- receiving an ultrasound image comprising at least one blood vessel;
- generating a user-movable color box in the ultrasound image;
- generating a user-movable watermark within the user-movable color box in the ultrasound image; and
- designating a blood vessel of interest from the at least one blood vessel based on a proximity of the watermark to one of the blood vessels.

11. The method of claim 10 further comprising:

- adjusting an adaptive imaging system parameter based on the designated blood vessel of interest.

12. The method of claim 10 or 11, wherein the user-movable color box and the user-movable watermark are user-movable in synchronism.

13. The method of any of claims 10 to 12, wherein the adaptive imaging system parameter comprise color Doppler imaging parameters, wherein the color Doppler imaging parameters are preferably any one or a combination of: transmit Frequency, Color Box Steering, Color Scale, Gain, Frequency, Wall Filter, Persistence, Smoothing, Dynamic Range and Grayscale Priority.

14. The method of any one of claims 10 to 13 further comprising:

- receiving a user input to move the user-movable color box and/or the user-movable watermark on the ultrasound image.

15. A computer program product comprising instructions, which when carried out by a processor, cause the processor to carry out the method according to any of claims 1 to 5.

FIG. 1

FIG. 2

FIG. 3

FIG. 4

FIG. 5

FIG. 6A

FIG. 6B

FIG. 7

FIG. 8

Europäisches
Patentamt

European
Patent Office

Office européen
des brevets

# EUROPEAN SEARCH REPORT

Application Number

EP 24 19 8482

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 050 515 B1 (SAMSUNG MEDISON CO LTD [KR]) 17 May 2023 (2023-05-17) | 1,4,6,9 | INV.<br>A61B8/00 |
| Y | * paragraph [0001] * | 2,3,5,7 | G01S7/52 |
| A | * paragraph [0031] *<br>* paragraph [0138] *<br>* figure 9 * | 8,10-15 | |
| | ----- | | |
| Y | US 2014/221838 A1 (LOUPAS THANASIS [GR] ET AL) 7 August 2014 (2014-08-07) | 2,3,5 | |
| A | * paragraph [0027] *<br>* paragraph [0026] * | 8,10-15 | |
| | ----- | | |
| Y | US 2021/401405 A1 (GURACAR ISMAYIL M [US] ET AL) 30 December 2021 (2021-12-30) | 7 | |
| A | * figures 5A, 5B *<br>* paragraph [0057] * | 8,10-15 | |
| | ----- | | |

**TECHNICAL FIELDS SEARCHED (IPC)**

G01S
A61B

The present search report has been drawn up for all claims

| Place of search | Date of completion of the search | Examiner |
|---|---|---|
| Munich | 28 January 2025 | Ordavo, Ivan |

EPO FORM 1503 03.82 (P04C01)

## ANNEX TO THE EUROPEAN SEARCH REPORT
## ON EUROPEAN PATENT APPLICATION NO.

EP 24 19 8482

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2025

| Patent document cited in search report | | Publication date | Patent family member(s) | | Publication date |
|---|---|---|---|---|---|
| EP 3050515 | B1 | 17-05-2023 | EP | 3050515 A1 | 03-08-2016 |
| | | | KR | 20160093487 A | 08-08-2016 |
| | | | US | 2016220231 A1 | 04-08-2016 |
| US 2014221838 | A1 | 07-08-2014 | CN | 103842841 A | 04-06-2014 |
| | | | EP | 2745137 A1 | 25-06-2014 |
| | | | JP | 5992044 B2 | 14-09-2016 |
| | | | JP | 2014528266 A | 27-10-2014 |
| | | | MX | 346426 B | 21-03-2017 |
| | | | RU | 2014117544 A | 10-11-2015 |
| | | | US | 2014221838 A1 | 07-08-2014 |
| | | | WO | 2013046087 A1 | 04-04-2013 |
| US 2021401405 | A1 | 30-12-2021 | CN | 113842157 A | 28-12-2021 |
| | | | DE | 102021116225 A1 | 30-12-2021 |
| | | | KR | 20220000813 A | 04-01-2022 |
| | | | US | 2021401405 A1 | 30-12-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5997479 A, Savord
- WO 6013032 A, Savord
- WO 6623432 A, Powers
- US 5833613 A, Averkiou
- US 6443896 B, Detmer
- US 6530885 B, Entrekin
- US 5315999 A, Kinicki
- US 11744557 B, Holmes